# EUROPEAN PATENT APPLICATION

(11) **EP 2 637 015 A1**
(43) Date of publication of application: **11.09.2013**
(21) Application number: 11837816.5
(22) Date of filing: 16.09.2011
(51) Int. Cl.: G01N 15/14, G01N 21/27, G01N 21/64, G01N 33/48, G01N 33/483

(54) **CELL ANALYZER**

(30) Priority: 01.11.2010 JP 2010245093
(71) Applicant: Kanagawa Academy Of Science And Technology, Kawasaki-shi, Kanagawa 213-0012 (JP); National University Corporation Tokyo Medical and Dental University, Bunkyo-ku Tokyo 113-8510 (JP); On-Chip Cellomics Consortium, Tokyo 100-0005 (JP); Kanagawa Prefectural Hospital Organization, Kanagawa 231-0005 (JP); Kinki University, Higashiosaka-shi Osaka 577-8502 (JP)
(72) Inventor: YASUDA Kenji, Tokyo 113-8510 (JP); TERAZONO Hideyuki, Kawasaki-shi Kanagawa 213-0012 (JP); KIM Hyonchol, Kawasaki-shi Kanagawa 213-0012 (JP); HAYASHI Masahito, Kawasaki-shi Kanagawa 213-0012 (JP); HATTORI Akihiro, Tokyo 100-0005 (JP); NISHIO Kazuto, Osakasayama-shi Osaka 589-8511 (JP); ARAO Tokuzo, Osakasayama-shi Osaka 589-8511 (JP); MIYAGI Yohei, Yokohama-shi Kanagawa 241-0815 (JP); OHTSU Takashi, Yokohama-shi Kanagawa 241-0815 (JP)
(74) Representative: Prinz & Partner
(86) International application number: PCT/JP2011/071258
(87) International publication number: WO 2012/060163

(57) **Abstract**

Provided is a cell concentration/purification device having a function of successively locating cells in a specific area of a microchannel, and a function of sequentially capturing cell images by use of light from a plurality of monochromatic light sources on an image basis, and performing comparative analysis of the cell images to recognize individual cells based on information on the shape of the cells and an absorption spectral distribution of the cells or inside of the cells, thereby selectively separating/purifying the cells.

## Description

### TECHNICAL FIELD

The present invention relates to a cell analysis device.

### BACKGROUND ART

In biological tissues in multi-cell organisms, various cells each play its own role to keep the function thereof in a coordinated manner. When a part of the cell becomes a cancer cell (herein, the term "cancer" encompasses cancers and tumors), the part becomes a neoplasm different from the area around the part. Such a cancer area and a normal tissue area far from the cancer area are not necessarily separated along a border, and the area around the cancer area is influenced in some way. Therefore, in order to analyze the function of an organ tissue, a small number of cells present in a narrow area needs to be separated in a short time, as simply as possible, and with minimum loss.

In the field of regenerative medicine, there is an attempt to separate a stem cell of an organ from the tissue and re-culture the stem cell for differentiation-induction in order to regenerate a target tissue and finally regenerate an organ.

For identifying or separating a cell, some index is needed. In general, cells are distinguished as follows.
1) Morphological cell classification by visual observation: this is used, for example, for inspecting a bladder cancer or a urethra cancer by inspection of heterocysts appearing in the urine, classification of heterocyst in the blood, cancer inspection by cytodiagnosis on the tissue.
2) Cell classification by cell surface antigen (marker) staining using a fluorescent antibody test: A cell surface antigen generally called a "CD marker" is stained with a fluorescence labeled antibody specific thereto. This is used for cell separation or flow cytometry by use of a cell sorter or cancer inspection by use of tissue staining. Needless to say, this method is widely used for cytophysiological studies and industrial use of cells as well as for medicine.
3) In an example of stem cell separation, target stem cells are separated as follows.
   Cells including stem cells are roughly separated using a fluorescent dye introduced into the cells as a reporter, and then the cells are actually cultured. Since no effective marker for stem cells has been established, the cells are actually cultured and only the differentiated-induced cells are utilized to substantially separate the target cells.
4) Minute organelles in the cells and intracellular antibiotic substances have different light absorption characteristics and birefringence characteristics in accordance with the type thereof. As technologies utilizing this to capture an image of these minute organelles or intracellular antibiotic substances and observe the state thereof in the cells, phase optical microscopy and differential interference optical microscopy are available. When light components of a plurality of wavelengths are used at the same time with these microscopes, an image is blurred due to chromatic aberration. Therefore, monochromatic light is used for the observation.
5) There are staining methods for specifically staining a specific cell or intercellular organelle. For example, according to the Gram staining, the cell or intercellular organelle is stained crystal violet or gentian violet, subjected to mordanting with an iodine solution, then decolored with alcohol, and then counterstained with fuchsine or safranin. As a result, the Gram-positive bacteria are stained dark blue or bluish violet and the Gram-negative bacteria are stained red or pink by counterstaining. Therefore, by identifying such a color of the cells, it can be determined whether the bacteria are Gram-positive or Gram-negative. According to the Romanowsky staining, staining is performed with a combination of reduced eosin and methylene blue (may occasionally contain azure A and azure B, which are oxides thereof). This is used for examining a sample of bone marrow biopsy, bone marrow aspirate or peripheral blood smear. By use of this method, leukocytes of different types can be easily distinguished. According to the Papanicolaou staining, the nucleus is stained with hematoxylin, the cytoplasm is stained with orange G, eosin Y and light green SF (in the order from a dye having a smaller molecular weight), and the lipoid is stained with basic brown. A cytoplasm having a fine structure easily allows a dye having a smaller molecular weight to be incorporated therein (e.g., keratinizing type squamous cell: orange), and a cytoplasm having a rough structure allows both of a dye having a small molecular weight and a dye having a large molecular weight to be incorporated therein. A dye having a large molecular weight has a low mobility and a large electric charge, and therefore is easily adsorbed into the cell (e.g., non-keratinizing type squamous cell, grandular cell: light green). Thus, malignant tumor cells and infectious diseases are identified by the difference in colors from dark bluish green to dark red. The Giemsa staining uses a mixture solution of methylene blue, which is a basic dye, methylene azure, which is an oxidized substance thereof, and eosin, which is an acidic dye. These dyes have different staining capabilities in accordance with methyl groups at the terminus or the influence of molecules permeating into the cells. Azure eosinate, which has a relatively small number of methyl groups, permeates into the cytoplasm and also the nucleus, and is bonded with a phosphoric acid group in the nucleic acid, and thus exhibits a reddish purple tone. By contrast, methylene blue, which has a relatively large molecule and a strong polarity, is bonded with protein in the cytoplasm and thus exhibits a bluish tone. Based on a degree of staining, information on the cells can be obtained. What is important here is that existing methods of identifying cells by staining is a measuring method using the intuition of the observer and the target thereof is a spectrum of the entire visible light spectrum. In more detail, according to the existing methods, a plurality of dyes having different absorption characteristics are combined. Depending on the degree of color development (absorption) of each of the dyes combined, namely, depending on the visual strength of each of the color components combined, the color which is visually perceived varies. The existing methods rely on processing of such visual information. This is a largest difference from the fluorescence measurement/evaluation technology, by which quantitative measurement is performed on fluorescence of a specific one wavelength.

It is important for biological and medical analyses to separate and recover specific cells in the cultured solution as described above. For separating cells by the difference in the specific gravity of the cells, velocity sedimentation is usable. However, in the case where there is almost no difference in the specific gravity, for example, in the case where unsensitized cells and sensitized cells are to be distinguished, the cells need to be separated one by one based on information obtained by staining with fluorescent antibody or information obtained by visual checking.

For such a technology, a cell sorter, for example, is available, which is operated as follows. After cells are treated with fluorescence staining, each cell is isolated and caused to be contained in a charged droplet, and the droplets are dripped one by one. While the droplets are falling down, a high electric field is applied in an optional direction in a planar direction normal to the falling direction, based on whether or not there is fluorescence in the cell in the droplet and the magnitude of light scattering, so that the falling direction is controlled. Thus, the cells are fractionated into a plurality of containers located below and recovered. (Non-patent Document 1: Kamarck, M. E., Methods Enzymol., Vol. 151, pp. 150-165 (1987))

However, this method has the problems that it costs high; the device is large; a high electric field of several thousand volts is necessary; a large amount of samples concentrated to a certain concentration are necessary; there is a risk that the cells may be damaged on the stage of creating the droplets; samples cannot be directly observed; and the like. In order to solve these problems, a cell sorter including microchannels formed by use of a microprocessing technology, such that cells flowing in a layered flow in the channel are separated while being directly observed with a microscope, has been developed (Non-patent Document 2: Micro Total Analysis, 98, pp. 77-80 (Kluwer Academic Publishers, 1988; Non-patent Document 3: Analytical Chemistry, 70, pp. 1909-1915 (1988)). However, with this cell sorter formed by use of the microprocessing technology, the response speed of sample separation to the observation means is low. In order to use this cell sorter practically, a separation method providing a higher response speed without damaging the samples is needed. There are also the following problems. The cell concentration of the sample solutions to be used needs to be increase to a certain level in advance; because when the cell concentration is low, the separation efficiency of the device cannot be sufficiently raised. In the case where trace amounts of samples are concentrated in another device, it is difficult to recover the concentrated solutions with no loss, and problems undesirable for reusing the recovered cells occur, for example, the problem that the cells are contaminated on a complicated pre-processing stage.

In order to solve these problems, the present inventors developed a cell analysis/separation device utilizing a microprocessing technology. This cell analysis/separation device fractionizes samples based on the minute structure of the samples and the fluorescence distribution in the samples, and thus can analyze and separate the sample cells in a simple manner without damaging the samples (Patent Document 1: Japanese Laid-Open Patent Publication No. 2003-107099; Patent Document 2: Japanese Laid-Open Patent Publication No. 2004-85323; Patent Document 3: WO2004/101731).

Today, the level of detection of cancer tissues has been remarkably raised by improvement of MRI (magnetic resonance imaging) and CT (computed tomography). For identifying whether the tumor is malignant or benign, there is no technique exceeding the evaluation by use of biopsy. A known problem of a malignant tumor is that the tumor is spread to another organ by an ability of infiltrating from the tissue of the cancer cell itself into the blood vessel or lymphatic vessel. Such malignant tumor cells circulating in the peripheral blood are called "circulating tumor cells (CTCs), and it is considered that about several hundred cancer cells are present in 100,000 blood cells (including erythrocytes). Recently, anticancer medicines against specific targets have been developed one after another. Therefore, once the type of the malignant tumor in the blood is identified, an anticancer medicine destroying the cells effectively can be selected.

In the meantime, regarding an imaging method capable of classifying the cells more precisely based on an image of cells, quantitative imaging cytometry has recently been studied (Non-patent Document 4: Harnett, M., "Laser scanning cytometry: understanding the immune system in situ. Nature Review Immunology, Vol. 7, pp. 897-904 (2007)). This imaging cytometry combines the concepts of the conventional imaging method performed by use of an optical microscope and cytometry. In addition to the conventional imaging performed randomly on tissues by use of a microscope, a reference value is set for a specific index such as the cell shape, fluorescent labeling or the like, and all the cells in the tissue are measured to provide statistics. In this method, for fluorescence measurement or the like, a scanning fluorescence imaging method of scanning converged laser light for measurement is mainly used.

In the meantime, as a technology of acquiring a plurality of fluorescence images of the entire cell image or acquiring a plurality of fluorescence polarization images on a light receiving surface of one camera at the same time, dual-view microscopy is available (Non-patent Document 5: Kinoshita K Jr., Itoh H, Ishiwata S, Hirano K, Nishizawa T, Hayakawa T, "Dual-view microscopy with a single camera: real-time imaging of molecular orientations and calcium.", J Cell Biol: 1991 Oct; 115(1): 67-73). According to this, light from a light source for phase microscopic observation and excited light for fluorescence observation are introduced onto the same optical path to observe the samples. Then, wavelength division is performed by a dichroic mirror to project a fluorescent image and a phase image respective on two halves of a light receiving surface of one CCD camera. Thus, the fluorescence image and the phase image are recorded in one frame at the same time. By such a combination, two fluorescent images can be observed, or a longitudinal fluorescence polarization image and a transverse fluorescence polarization image may be compared, for example. However, this technology is not used for absorption spectrum measurement or the like.

### CITATION LIST

### PATENT LITERATURE

Patent Document 1: Japanese Laid-Open Patent Publication No. 2003-107099
Patent Document 2: Japanese Laid-Open Patent Publication No. 2004-85323
Patent Document 3: WO2004/101731.

### NON-PATENT LITERATURE

Non-patent Document 1: Kamarck, M. E., Methods Enzymol., Vol. 151, pp. 150-165(1987)
Non-patent Document 2: Micro Total Analysis, 98, pp. 77-80 (Kluwer Academic Publishers, 1988)
Non-patent Document 3: Analytical Chemistry, 70, pp. 1909-1915 (1988)
Non-patent Document 4: Harnett, M., "Laser scanning cytometry: understanding the immune system in situ. Nature Review Immunology, Vol. 7, pp. 897-904 (2007)
Non-patent Document 5: Kinoshita K Jr., et al., Dual-view microscopy with a single camera: real-time imaging of molecular orientations and calcium. J Cell Biol. 1991 Oct; 115(1):67-73

### SUMMARY OF INVENTION

### TECHNICAL PROBLEM

Although it is considered important on the clinical level to check the presence of CTCs regarding the spread of cancer, a diagnostic standard using this as an index of spread of cancer has not been established yet. The reason for this is as follows. The CTCs are diversified and rare. Therefore, in order to specify that cancer cells of diversified shapes obtained from the blood as being cancer cells merely based on the shapes thereof, a huge amount of shape databases need to be constructed. Therefore, it is very difficult to develop an algorithm for selectively recovering cancer cells with certainty.

Therefore, conventionally, cancer cells in the blood are identified by fluorescent labeling. However, there is no technique for guaranteeing that fluorescent labeling specific to a specific cancer is appropriately selected. Because of this situation, CTC measurement involves a problem of providing a "false negative" conclusion that cancer cells, although being present in the blood, cannot be detected.

Therefore, if a technology of monitoring CTCs flowing in the blood is realized, such a technology can measure, quantitatively, the presence of malignant tumor cells flowing in the blood, which cause cancer spread, evaluate the effect of the administered anticancer medicine quantitatively and continuously, and thus prevent unnecessary or excessive administration of an anticancer medicine, and also detect presence/absence of recurrence, for the first time in the world.

### SOLUTION TO PROBLEM

In this situation, the present inventors provides a cell analysis device capable of identifying cells based on absorption spectral imaging data of the cells and inside of the cells and selectively recovering target cells when necessary. This cell analysis device is obtained as a result of combining flow cytometry and cell sorting based on the cell shape or cell population imaging observation technology already proposed by the present inventors, a technology of recovering cells by comparative analysis performed by simultaneous fluorescence detection, and an absorption spectral analysis technology for cell imaging, which is an existing cell inspection and identification technology. According to the conventional flow cytometry, when a signal is detected from a sample in the absence of background light such as fluorescent light, scattered light or the like, an optical signal can be acquired from trace amounts of cells in a bulk solution container. However, with the conventional technology, minute decrease of incident light caused by absorption of light by the trace amounts of cells present in the bulk container cannot be quantitatively measured in a bulk, or absorption spectrum of a specific area of the cell of inside of the cell cannot be measured with spatial resolution. Therefore, the present inventors newly combined a super high speed camera and flash monochromatic light sources to perform imaging at each of wavelengths of the incident light and thus to acquire an absorption spectrum having spatial resolution. Thus, the present inventors newly propose an absorption flow cytometry technology.

Some of cancer cells, stem cells and the like flowing in the blood other than erythrocyte cells and leukocyte cells are diversified and/or undifferentiated and therefore may not be identified and handled by merely staining or antibody reaction. For such cells, a technique of staining the known blood cells such as erythrocyte cells, leukocyte cells and the like and recovering the cells which are not stained as the target cells, a technique of recovering cells having different shapes from the known shapes of cells, or the like is effective, as well as the technique of labeling the cells. Namely, the present inventors combined flow cytometry and cell sorting based on the cell shape or cell population imaging observation technology already proposed by the present inventors, a technology of recovering cells by comparative analysis performed by simultaneous fluorescence detection, and an absorption spectral analysis technology for cell imaging, which is an existing cell inspection and identification technology; and thus provide a technology of identifying cells based on absorption spectral imaging data of the cells and inside of the cells. The present inventors newly propose a method of using this technology for labeling the known erythrocyte cells and leukocyte cells in the blood, and recovering the cells which are not labeled as candidate cells for cancer cells or stem cells. For identifying cells by shape, immature cells having a very high ratio of the nucleus size with respect to the size thereof may be especially distinguished and recovered.

Namely, the present invention provides the following device.
[1] A cell analysis device for measuring cells in a sample cell solution containing the cells derived from a subject to selectively separate/purify the cells, the cell analysis device comprising:
   a cell sorter chip including a first channel for allowing the sample cell solution containing the cells including target cells to flow therein, a cell detection section for detecting an image, obtained by absorption spectral imaging, of the cells in the first channel, and a cell separation section for separating the target cells from the other cells and selectively recovering the target cells, the cell separation section including at least two branched channels branched from the first channel;
   an optical system including light directing means for directing light from a plurality of monochromatic light sources located to form a visible light spectrum to the cells flowing in the first channel, and a high speed camera for capturing an image of the cells as an image of each of monochromatic light components at an image capturing rate of at least 200 frames/second, the high speed camera capable of comparing the images of the monochromatic light components to distinguish absorption characteristics of the cells by use of spatial resolution; and
   a mechanism for selectively applying, to the cells, an external force for guiding the target cells to either one of the at least two branched channels.
[2] The cell analysis device according to [1], wherein the external force is an ultrasonic radiation pressure, gravity, an electrostatic force, or a dielectric electrophoretic force.
[3] The cell analysis device according to [1] or [2], which combines images of the cells obtained at the wavelengths to estimate a visually observed color, detects each of the cells on the level of one cell based on a result of spatial distribution in the cells, and thus identifies the cells to be the target cells.
[4] The cell analysis device according to any one of [1] through [3], which binarizes an image, obtained by optical imaging, of the entirety of the cells, detects each of the cells on the level of one cell by use of at least one index selected from the group consisting of luminance center of gravity, area size, circumferential length, longer diameter and shorter diameter of the binarized image, and thus identifies the cells to be the target cells.
[5] The cell analysis device according to [4], wherein the cells in the sample cell solution are labeled with fluorescence, the optical system further includes a fluorescence detector, and information on a fluorescence image of the cells is used as an additional index.
[6] A cell analysis device for measuring cells in a sample cell solution containing the cells derived from a subject to detect the cells, the cell analysis device comprising:
   a channel chip including a channel for allowing the sample cell solution containing the cells including target cells to flow therein, and a cell detection section for detecting an image, obtained by absorption spectral imaging, of the cells in the channel; and
   an optical system including light directing means for directing light from a plurality of monochromatic light sources located to form a visible light spectrum to the cells flowing in the first channel, and a high speed camera for capturing an image of the cells as an image of each of monochromatic light components at an image capturing rate of at least 200 frames/second, the high speed camera capable of comparing the images of the monochromatic light components to distinguish absorption characteristics of the cells by use of spatial resolution.
[7] The cell analysis device according to any one of [1] through [6], comprising the optical system including the light directing means for directing light from a plurality of monochromatic light sources located to form a visible light spectrum to the cells flowing in the channel, a slit for narrowing a resultant image so as to divide a light receiving surface of the image capturing camera into a number of spectra which are to be acquired, a combination of two or more dichroic mirrors and two or more optical filters capable of equally dividing an image spectrum, and the high speed camera for capturing an image of the cells as an image of each of monochromatic light components at an image capturing rate of at least 200 frames/second, the high speed camera capable of comparing the images of the monochromatic light components to distinguish absorption characteristics of the cells by use of spatial resolution.
[8] A cell analyzing method for measuring cells in a sample cell solution containing the cells derived from a subject to selectively separate/purify the cells, the method comprising the steps of:
   introducing the sample cell solution into a first channel of a cell sorter chip, which includes the first channel for allowing the sample cell solution containing the cells including target cells to flow therein, a cell detection section for detecting an image, obtained by absorption spectral imaging, of the cells in the first channel, and a cell separation section for separating the target cells from the other cells and selectively recovering the target cells, the cell separation section including at least two branched channels branched from the first channel;
   directing light from a plurality of monochromatic light sources to the cells flowing in the first channel;
   capturing an image of the cells by use of a high speed camera capable of capturing an image of the cells as an image of each of monochromatic light components at an image capturing rate of at least 200 frames/second and comparing the images of the monochromatic light components to distinguish absorption characteristics of the cells by use of spatial resolution;
   selectively applying, to the cells, an external force for guiding the target cells to either one of the at least two branched channels, based on a result of analysis of the captured image; and
   recovering the target cells sorted and guided to either one of the branched channels.
[9] The method according to [8], wherein the cells in the sample cell solution are labeled with an antibody or a dye in advance.
[10] The method according to [8] or [9], wherein as the sample cell solution, a sample cell solution containing cells sorted by the cell shape and/or staining is used.
[11] A cell analysis device for identifying cells in a sample cell solution containing the cells derived from a subject, the cell analysis device comprising:
   a pulsed light source including a monochromatic light source array of a plurality of monochromatic light sources corresponding to continuous specific wavelength bands covering a wavelength range from 280 nm to 780 nm, the pulsed light source being controllable by a control program such that each of the monochromatic light sources emits pulsed light;
   a cell holding section for holding the sample cell solution containing the cells including target cells, the cell holding section being formed of an optically transparent material to the wavelength range corresponding to the monochromatic light source array;
   an image capturing camera having a wavelength characteristic with which an image of the cells can be captured at the wavelength range corresponding to the monochromatic light source array;
   a recording section in which the captured image is recorded; and
   an image processing section for recording the image in the recording section in a state where a wavelength of each monochromatic light component from the pulsed light source and a frame of the captured image are correlated to each other, and using a result of the recording to compare the images of the monochromatic light components to distinguish absorption characteristics of inside of the cells by use of spatial resolution.
[12] The cell analysis device according to [11], wherein the pulsed light source includes the array of monochromatic pulsed light sources capable of emitting pulsed light in a time duration which is shorter than an image capturing time duration of one frame of the image capturing camera.
[13] The cell analysis device according to [12], comprising a high speed camera capable of capturing 10,000 frames per second as the image capturing camera and the array of monochromatic light LED light sources having an emission time less than 0.1 ms.
[14] The cell analysis device according to [11], which is programmed such that two or more of the monochromatic light sources of the monochromatic light source array emit pulsed light at the same time and thus the image captured by the image capturing camera is an image obtained by absorption of light of any two ore more wavelengths in accordance with the light emission from the monochromatic light sources.
[15] The cell analysis device according to [11], wherein the cell holding section formed of the optically transparent material to the wavelength range corresponding to the light source for observation is a flow cell having a function of allowing the sample cell solution to flow therein successively.
[16] The cell analysis device according to [15], wherein the flow cell having the function of allowing the sample cell solution to flow therein successively includes a cell separation/purification section for separating/purifying the cells and moving the target cells downstream with respect to an observation area.
[17] The cell analysis device according to [11], wherein the order of monochromatic light components directed from the monochromatic light source array is optionally set by the control program.
[18] The cell analysis device according to [11], which distinguishes the target cells using disappearance of a nucleus in a cell image, obtained from the captured image, as an index.
[19] The cell analysis device according to [11], which distinguishes the target cells using a ratio between the size of each cell and the size of the nucleus of the corresponding cell in a cell image, obtained from the captured image, as an index.
[20] The cell analysis device according to [11], which distinguishes the target cells using the degree at which the cells are gathered to form a population in a cell image, obtained from the captured image, as an index.

### ADVANTAGEOUS EFFECTS OF INVENTION

According to the present invention, it can be estimated what visual color is possessed by trace amounts of cells in a subject by absorption, absorption spectral characteristics can be analyzed, cells can be identified by the color or the intracellular absorption characteristic distribution, and target cells can be selectively recovered and purified, and thus correct genetic information and expression information of the target cells can be analyzed.

### BRIEF DESCRIPTION OF DRAWINGS

[FIG. 1] FIG. 1 is a schematic view conceptually showing an example of cell analysis device 1 according to the present invention.
[FIG. 2] FIG. 2 is a schematic view conceptually showing an example of cell sorter chip used in the cell analysis device 1 according to the present invention.
[FIG. 3] FIG. 3 schematically shows an example of imaging recording used in the present invention. '
[FIG. 4] FIG. 4 schematically shows an optical system and a second example of imaging recording used in the present invention.
[FIG. 5] FIG. 5 shows an example of cell images obtained by absorption of monochromatic light of a specific wavelength according to the present invention.

### DESCRIPTION OF EMBODIMENTS

Hereinafter, embodiments of the present invention will be described in more detail with reference to the drawings. The following embodiments are merely illustrative, and the present invention is not limited to these embodiments.

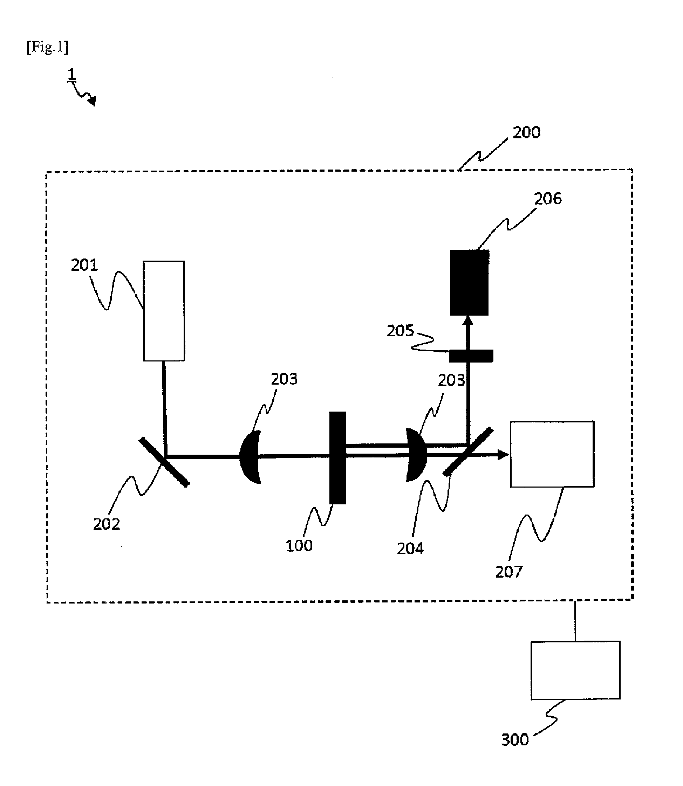

FIG. 1 is a conceptual view of a cell analysis device 1 in an embodiment according to the present invention.

As shown in FIG. 1, the cell analysis device 1 according to the present invention typically includes an image detection type single-cell separation/purification module 200 for acquiring image data of about 10,000 cell images per second of cells flowing in a microchannel which is formed in a substrate of a cell sorter chip 100, and purifying and recovering cell populations or cell clusters by a processing capability of 10,000 cells per second at the maximum in real time based on the result of analyzing the image information; and a recording/control section 300 for recording/analyzing various data acquired by the module 200 to control various sections of the module 200. The cell analysis device 1 according to the present invention may include a display for displaying the acquired image data, analysis results and the like although such a display is not shown.

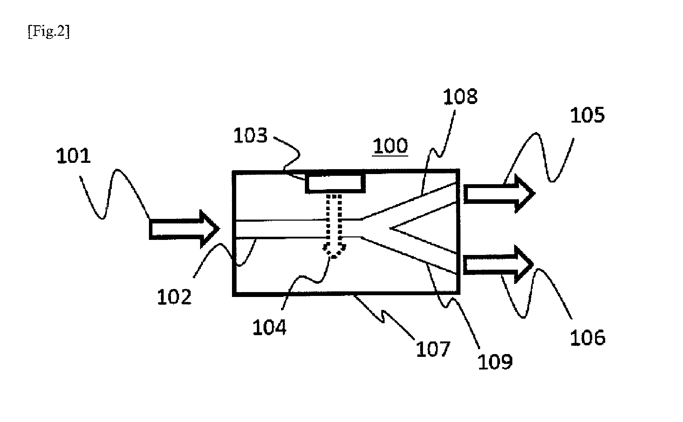

FIG. 2 is a schematic view showing an example of cell sorter chip 100 for separating cells to be used in the cell analysis device 1 according to the present invention. As shown in FIG. 2, the cell sorter chip 100 includes a channel 102 formed in a chip substrate 107, an external force generator 100 for applying an external force to cells flowing in the channel 102, and branched channels (108, 109) to which the separated cells flow. The external force generator 103 may not necessarily be integral with the chip substrate 107.

The cell sorter chip 100 used in the cell analysis device 1 according to the present invention typically includes, in the channel 102 of the chip substrate 107, a cell separation/purification section and an optical analysis section (or cell detection section) provided on a stage before the separation section. The cell separation/purification section has a function of separating/purifying the cells, and the optical analysis section distinguishes the cells to be separated/purified. In the case where the cells are merely to be identified by a flow cytometry function, it is sufficient that only the cell detection section is included, and the cell separation/purification section may not be necessary.

The cell separation/purification section includes at least two branched channels (108, 109). External forces are applied to the cells flowing in the channel in different directions. In order to guide cells to be recovered to one of the two branched channels and guide the remaining cells to the other channel, external forces are applied to these cells in opposite directions from each other to move the cells to different positions. Thus, the cells can be guided to either one of the two branched channels.

In order to measure the absorption of the cells by flow cytometry, a function of imaging the cells is needed. The cell analysis device 1 according to the present invention has a function of precisely identifying and evaluating shapes of the cells based on an image acquired by the cell detection type single-cell separation/purification module, and therefore can distinguish the shapes of the cells and recover trace amounts of cells from the sample cells successively transported in the channel even if the cells are not stained, in a state where the loss of the cells by contamination or operation is minimized. Hereinafter, various sections of the cell detection type single-cell separation/purification module 200 will be described specifically.

Referring to FIG. 1, the cell detection type single-cell separation/purification module 200 typically includes the cell sorter chip 100, a light source 201, a mirror 202, a light collection lens 203, a dichroic mirror 204, and optionally, a filter 205, a high sensitivity light detector (or fluorescence detector) 206, and a high speed camera 207.

The light source 201 is formed of, for example, a light source array for spectral analysis, which is a set of pulse lasers or high luminance LEDs each for emitting monochromatic light toward the cells passing the cell sorter chip 100. Preferably, the light source 201 is formed of, for example, a monochromatic light source array for spectral analysis, which is a combination of a plurality of monochromatic pulse lasers or monochromatic high luminance LEDs each for emitting monochromatic light and acting as a light source set for covering a wide wavelength range. The light sources may emit continuous light, but preferably generate pulsed light in synchronization with a shutter cycle of the high speed camera 207 in order to improve the spatial resolution of an image with less blur. Owing to this, an image having a higher time resolution is obtained by light radiation of a shorter time.

According to the present invention, a plurality of monochromatic light sources are combined, so that an image having layers of images taken with two (or more) light sources, which is not acquired by a conventional device using a band-pass filter, can be acquired. Especially in the case where each of monochromatic light sources emits pulsed light instead of continuous light, an absorption spectral image can be acquired as a result of combining absorption images obtained by the plurality of monochromatic light sources for a plurality of wavelengths. In a preferable embodiment, the light sources each act as a pulsed light source for emitting pulsed light at an interval of a time duration shorter than the shutter cycle (image capturing time resolution for capturing one frame) of the high speed camera 207 for acquiring an image. A conventional xenon flash light source emits light about 400 times per second at the maximum. Based on this, a high speed camera capable of capturing, for example, 10,000 frames per second needs a light source which emits light at an interval of at least 0.1 ms or shorter. According to the state of the art, as a light source capable of emitting any monochromatic light at such a short interval, a monochromatic pulsed light LED is available, but such a light source is not limited to this. A plurality of images (image frames) are captured with monochromatic pulsed light components corresponding to different parts of a wavelength range, which is a target of acquisition of an absorption spectral image. As a result of comparing the captured plurality of images, an absorption spectral image of this wavelength range is obtained. Namely, for example, when monochromatic pulsed light of 280 nm to 330 nm is emitted, an image capturing camera captures one image; and then the next monochromatic light (e.g., 330 nm to 380 nm) is emitted within a frame time duration in which the image capturing camera captures another image, to acquire an absorption image in this wavelength range. This series of operations is repeated. As a result, a set of the captured images are combined to form an absorption spectral image having spatial information.

The high sensitivity light detector (or fluorescence detector) 206 is formed of a photomultiplier for detecting fluorescence or the like.

The high speed camera 207 may typically include means for adjusting the intensity of light from the light source of each wavelength in accordance with the characteristics of the light receiving side so that the output of the light receiving side is approximately the same as the output of the light emission side. This adjustment is performed in order to adjust the wavelength dependence of the photoelectric conversion efficiency of a light receiving element which forms a light receiving face of the camera. Alternatively, the high speed camera 207 may be a camera including an electronic two-dimensional image capturing element, a computer or an amplification electronic circuit, for example, a CCD camera (video camera, digital camera) or the like which performs as follows. The light sources of different wavelengths are set to emit light of the same intensity, and receiving data regarding each wavelength is processed with amplification correction by use of an electronic circuit or an image processing program in consideration of the wavelength dependence of the photoelectric conversion efficiency of the light receiving element.

As a system for realizing image capturing, the device according to the present invention includes, in an embodiment, (1) a system for identifying the type of cells as follows. As the light sources, a plurality of monochromatic flash light sources are used. Light from each of the different monochromatic flash light sources is used to capture one image, and the cell images thus captured at a plurality of continuous wavelength ranges are subjected to comparative analysis. As a result, an image obtained by imaging and reflecting an absorption wavelength characteristic of the cells is acquired. The absorption wavelength characteristic is converted into (analogized as) a spectral characteristic or visual color information having a spatial distribution estimated by a combination of intensities of the absorbed (or transmitted) light having the wavelengths. Thus, the type of cells is identified. Such radiation of light from the pulsed light sources, image capturing, analysis and the like can be realized by a program by use of a computer (e.g., PC). Preferably, the light sources may include a plurality of monochromatic flash light sources of different wavelengths.

Now, each of the monochromatic flash light sources of the light source array may emit light of a wavelength range width of, for example, 50 nm in the range of 280 nm to 780 nm. Namely, for example, a light source for emitting monochromatic light of only the wavelength range of 280 nm to 330 nm, a light source for emitting monochromatic light of only the wavelength range of 330 nm to 380 nm, a light source for emitting monochromatic light of only the wavelength range of 380 nm to 430 nm, and the like are used. A total of 10 such monochromatic light sources are combined to form a monochromatic light source array covering the entirety of the above-mentioned wavelength range. In this example, the wavelength range width is, for example, 50 nm. It is preferable to appropriately select any of the wavelength range width from 10 nm to 100 nm (e.g., every 10 nm, every 20 nm, every 30 nm, etc.) in accordance with the purpose in consideration of the balance between the wavelength resolution and the analysis speed (number of monochromatic light sources for covering the entire wavelength range).

According to the present invention, when the light source is a monochromatic light source array, each of the light sources emits monochromatic light. Therefore, as long as the wavelength range which can be captured by the image capturing camera covers the entire wavelength range of the monochromatic light source array, it is not especially necessary to provide the image capturing camera with a filter to select a wavelength range of the transmitted light. Light which has passed an objective lens can be entirely guided to the camera. Therefore, an optical system of a simplest structure with no absorption band filter being required can be realized (namely, flash light from the monochromatic light source array is directed to the cells in the channel by a condenser lens, and the cell image obtained with monochromatic light is directly directed to an image capturing surface of the image capturing camera). In the case where a conventional light source covering a wide wavelength range is used, in order to obtain a plurality of monochromatic images providing substantially the same effect as that of the present invention, a complicated device structure of incorporating a plurality of band-pass filters corresponding to the wavelength bandwidths of the monochromatic images into the optical system is required. In addition, correction is required due to different transmittances of filters, and a problem that the transmitted light is attenuated by a combination of a plurality of filters needs to be dealt with. By contrast, by use of the above-described one feature of the present invention, a device structure which does not need any band-pass filter can be provided. As can be seen, according to the present invention, the above-described problem of the conventional art is solved. In addition, by merely and freely changing and adjusting the wavelength bandwidth for each of the monochromatic light sources included in the monochromatic light source array, images of wavelength bandwidths or areas for which optical filters are difficult to be formed by the conventional art can be obtained. Thus, spectral images of different wavelength bandwidths can be obtained easily. Unlike the conventional spectroscopy using a prism, slit or the like, absorption images of only a plurality of specific monochromatic light components to be paid attention to instantaneously can be successively acquired while other wavelength ranges are skipped. Alternatively, in order to make the light source array usable for protein or the like which has absorption characteristics thereof changed in accordance with the light directed thereto, for example, phytochrome of plants, the order of monochromatic light components to be directed can be freely changed by a program.

By use of the feature of the present invention, an image having layers of images taken with a plurality of monochromatic pulsed light components of any two or more wavelengths, which cannot be acquired by a conventional structure of a band-pass filter, can be acquired. Namely, a plurality of monochromatic pulsed light components of any two or more wavelengths in the wavelength range covered by the monochromatic light source array are directed at the same time. As a result, for example, an image having layers of absorption images of specific two monochromatic light components can be optically acquired as one image. Namely, for example, a monochromatic pulsed light component of 280 nm to 330 nm and a monochromatic pulsed light component of 480 nm to 530 nm are emitted at the same time, specifically, within one frame time duration of the image capturing camera. As a result, an image having absorption characteristics at the two wavelengths in combination, which is not obtained by the conventional device, can be acquired. Owing to this, one image which is a combination of absorption images of necessary wavelength ranges can be acquired instantaneously, especially for a target sample moving quickly. This provides a possibility that a spectral imaging field based on a new index of "layered spectroscopic imaging of a plurality of wavelengths", which is not existent conventionally, will be created as a new area of cell distinguishing.

In the above, the device structure is used for cell distinguishing performed by, specifically, a cell sorter. Alternatively, the device structure may be incorporated into an optical microscope, so that a layered absorption image of a plurality of monochromatic pulsed light components is captured by an image capturing camera. In the above example, a cell sorter having a cell separation function is described as an example of cell sorter. Alternatively, the cell sorter may be a substrate, a cell holding vehicle, a channel or the like having only a cell observation function without the cell purification function (or cell separation/purification section). For example, the cell sorter may be a substrate formed of a material such as, for example, synthetic quartz, which is optically transparent to the emission wavelength range of the above-described monochromatic pulsed light array, or a channel formed in such a substrate for allowing a cell-containing solution to flow therein. For example, the above-described device may be used for an absorption imaging cytometry technology of successively performing absorption imaging of samples flowing in the channel, needless to say.

In another embodiment, the device according to the present invention includes (2) a system for identifying the type of cells as follows. A plurality of monochromatic flash light sources or white light sources are used to divide an image of each wavelength range to be observed by a dichroic mirror or the like, and the divided images are projected on a light receiving surface of one CCD camera at the same time. At this time, the light receiving surface is equally divided into two, or in the case where images of a plurality of wavelengths are to be observed, the light receiving surface is divided into a number of the divided images to be observed. The images of the wavelength components which are projected are compared, and conversion is performed into (analogized as) a spectral characteristic or visual color information having a spatial distribution estimated by a combination of intensities of the absorbed (or transmitted) light having the wavelengths. Thus, the type of cells is identified.

In the case where white incident light or incident light having a mixture of a plurality of wavelengths is used, imaging data divided in accordance with the wavelength by use of a plurality of filters as described above may be projected on the respective divided areas of the light receiving surface of a single camera, so that spectral imaging data can be acquired.

By contrast, in the case where the plurality of monochromatic light sources emit light one by one, a technique of acquiring spectral imaging data substantially the same as above may be combined with a pulse light component from each monochromatic light source and one image obtained by imaging, so that spectral data can be acquired with no use of a filter. In this case, since a moving object is sequentially imaged by different monochromatic light components, position information needs to be corrected. However, since no wavelength filter is needed, there is an advantage that accumulated loss of light amount, which would be caused by use of many filters when, especially, wavelength resolution is performed many times for measurement, can be suppressed.

Needless to say, a process using an image and a process using fluorescence may be used in combination. However, when measurement by fluorescence is added, a filter 205 for fluorescence observation is additionally needed. Attention should be paid because absorption spectral imaging cannot be acquired for this wavelength. Although not shown, image data obtained by the high speed camera 207 may be displayed on a monitor of a computer (300) in real time so that the user can observe the data, in addition to be recorded. In the case where there are a plurality of fluorescence components to be observed, the filter 205 may be appropriately adjusted to transmit a plurality of excited light components, and a wavelength which does not overlap the wavelength of the fluorescence, to be detected on a later stage, may be is selected and directed to the cells. The dichroic mirror 204, the filter 205 and the fluorescence detector 206 may be assembled in accordance with the type of fluorescence, and a plurality of such assemblies may be combined. By use of such a structure, results of fluorescence observation on cell images can be used as data.

By use of a fluorescence measuring function of the cell analysis device according to the present invention, presence/absence of a fluorescence label in a target cell can be detected and confirmed on the level of one cell. The above-described absorption information and results of distinguishing of the fluorescence-labeled cells may be used together to make a comprehensive determination to selectively recover target cells. In this manner, according to the cell analysis device of the present invention, a plurality of pieces of information can be detected at the same time on the cells flowing in the channel of the cell sorter chip 100. Therefore, according to the cell analysis device of the present invention, cells can be precisely separated/purified based on an index which is not identifiable by a conventional scattered light detection type cell sorter technology.

The recording/control section 300 used in the cell analysis device 1 according to the present invention is formed of, for example, a personal computer including an electronic storage medium (e.g., ROM, RAM, hard disc, USB, memory card, etc.), a program type logical element, an SPGA or the like. The recording section and the control section may not be necessarily integral with each other.

An algorithm of cell recognition and separation used in the present invention has the following features.

In the case where cells are captured as an image for evaluation, an area where the cells are observed by a high speed camera such as a CCD camera or the like is provided in a part of the channel of the cell sorter chip 100. The measurement range is expanded in a planar manner, and cells are identified by image recognition and traced. Thus, cells are separated with more certainty. What is important at this point is the image capturing speed. With a general camera having a video rate of 30 frames/second, all the cells may not be captured in an image. A camera having a video capturing rate of at least 200 frames/second can usually recognize cells flowing in the channel at quite a high speed.

In the case where the image detection type single-cell separation/purification module 200 captures cells as an image for evaluation, an area where the cells are observed by a CCD camera is provided at a position upstream with respect to the branching position of the channel. When necessary, a cell separation area is provided downstream with respect thereto. Image data is acquired. In addition, laser light or the like is directed to the cells passing the channel, so that in the case where the cells are modified with fluorescence, the fluorescence can be detected by the light detector. In this case also, a branching point, which is the cell separation area, may be provided downstream with respect to the detection section.

In the case where the cells are separated in a sorting section, which is the cell separation area, the external force generator 103 may be used to apply an external force to the cells in the cell separation section from outside to move the cells. As the external force, for example, a dielectric electrophoretic force, an electrostatic force, an ultrasonic radiation pressure or the like is usable.

In the case where a dielectric electrophoretic force is used, a pair of comb-like electrodes are provided, and a channel for separating and discharging the cells is provided. In the case where an electrostatic force is used, a voltage is applied to electrodes to change the position of the cells in the channel. Cells are generally charged negative and therefore are moved toward the positive electrode. In the case where an ultrasonic radiation pressure is used as the external force, the cells may be guided to a sound pressure node.

Hereinafter, a cell analysis/separation method for separating/purifying target cells in sample cells including the target cells by use of the cell analysis device 1 according to the present invention will be described.

On the chip 100, the sample solution is first introduced in a direction of 101 from a flow inlet into the microchannel 102 by cell introduction means which does not generate a pulsating current caused by a syringe pump, air pressure, liquid level difference or the like (see FIG. 2). The cell-containing sample solution which has been introduced into the microchannel has cells measured and the type of the cells determined in a cell detection area located on a stage before the cell sorting section. Then, the sample solution passes either one of the two channels (108, 109) branched at the cell sorting/branching section in accordance with whether or not an external force is applied in a direction perpendicular to the flow from upstream to downstream by use of the external force generator 103, and thus is guided to either one of a first flow outlet 105 and a second flow outlet 106.

According to the present invention, for example, a pressure of introducing the sample cell solution into the chip can be used as a driving force for moving the liquid.

The image detection type single-cell separation/purification module 200 (see FIG. 1) checks whether there is fluorescence emission or not based on the fluorescence label on the level of one cell, as, for example, primary detection. Owing to this, whether each cell can be a target cell or not can be checked by a conventional labeling technology using fluorescence. Then, an image of a cell which emits fluorescence and thus can be a target cell is captured by the high speed camera 207 and is analyzed. Thus, information obtained from the image is analyzed as follows in accordance with the purpose. (1) the cell emitting fluorescence is checked by imaging regarding what absorption characteristics the cell has, or what is the absorption characteristic distribution in the cell is like. (2) It is distinguished, for example, whether the cell emitting fluorescence is in a healthy state or in an apoptosis state or the like in which the nucleus of the cell or the cell itself is deformed; namely, the shape of an intracellular organelle is distinguished. Alternatively, the size of the nucleus is compared against the size of the cell to distinguish the type of cancer cell. Still alternatively, it is distinguished whether or not there is an M phase cell in which the nucleus disappears. Based on such analysis results, healthy independent cells, cell populations or cell clusters in the blood, cells in an apoptosis state, or cells having a specific absorption characteristic can be recovered.

In the case where separation/purification is performed based on a determination made by use of an image detection result, the absorption characteristic of the cell, the absorption characteristic of a specific minute organelle in the cell, and also information such as the size difference of the cells, the number of cell populations and the like are acquired as image information. Based on the results, the cells are purified. For capturing an image, a high speed camera is used. In order to capture a high definition image, the emission from the light source is adjusted in accordance with the shutter cycle of the high speed camera, so that light is emitted from the light source only for a certain time duration in the time period when the shutter is released. For example, in the case where the shutter speed is 1/10,000 seconds, light is directed to the target cell from a light source controlled to emit light at high speed such as an LED light source, a pulsed laser light source or the like, only for 1/10 of 1/10,000 seconds. Thus, precise shape of the cells, for example, whether or not the sample cells are gathered to form a population or the like, can be acquired.

In the above example, the cells in the blood are mainly described as the target of detection, analysis and/or separation according to the present invention. However, the target is not limited to the cells in the blood, needless to say. For example, the target may be range from small bacteria to large animal cells (e.g., cancer cells). Especially among the cancer cells, cell populations or cell clusters may be the target, but the target is not limited to these cells. Therefore, the size of the sample cells is typically about 0.5 µm to about 100 µm. For this reason, when a channel having a cell separation function is formed in a surface of a biochip substrate and cell concentration and separation are successively performed, the first issue to consider is the width of the channel (cross-sectional shape). The channel is formed in one of the substrate surfaces in a space of about 10 to about 100 µm in a thickness direction and thus is formed substantially in a two-dimensional planar shape. In consideration of the size of the cells, a most typical size of the channel in the thickness direction is about 5 to about 10 µm for bacteria, and is about 100 to about 500 µm for cell populations or cell clusters. The sample cell solution can be prepared as follows. For, for example, inspecting the cells in the blood, human blood is directly sampled by use of a blood collection tube containing heparin and this can be used as a sample cell. Alternatively, in the case where it is wished to hemolyze and remove erythrocyte in advance, the erythrocyte may be treated with BD Pharm Lyse of a volume ten times as large as the volume of the erythrocyte at room temperature for 15 minutes and then used for analysis. In the case where any of various staining methods is used, a method used in conventional biopsy for each staining method is usable.

Regarding the procedure of recovering cancer cells, stem cells or the like, which are other than erythrocyte cells and leukocyte cells flowing in the blood by use of the above-described technology, in the case where the labeling antibody and/or the staining method is determined, the determined labeling antibody and/or the staining technology may be used as it is. However, there are cases where target cells are diversified and/or undifferentiated and therefore cannot be identified and handled by merely staining or antibody reaction. In such cases, leukocyte cells, erythrocyte cells and thrombocyte cells, which are blood cells already known, may be identified by the shape or stain, so that cells which are not identified by such a method can be recovered as the target cells. Namely, flow cytometry and cell sorting based on the above-described cell shape or cell population imaging observation technology proposed by the present inventors, a technology of recovering cells by comparative analysis performed by simultaneous fluorescence detection, and an absorption spectral analysis technology for cell imaging, which is an existing cell inspection and identification technology, may be combined to provide a technology of identifying cells based on absorption spectral imaging data of the cells and inside of the cells. This technology may be used for labeling the known erythrocyte cells and leukocyte cells in the blood, and the cells which are not labeled may be recovered as candidate cells for cancer cells or stem cells. For identifying cells by shape, immature cells having a very high ratio of the nucleus size with respect to the size thereof may be especially distinguished and recovered.

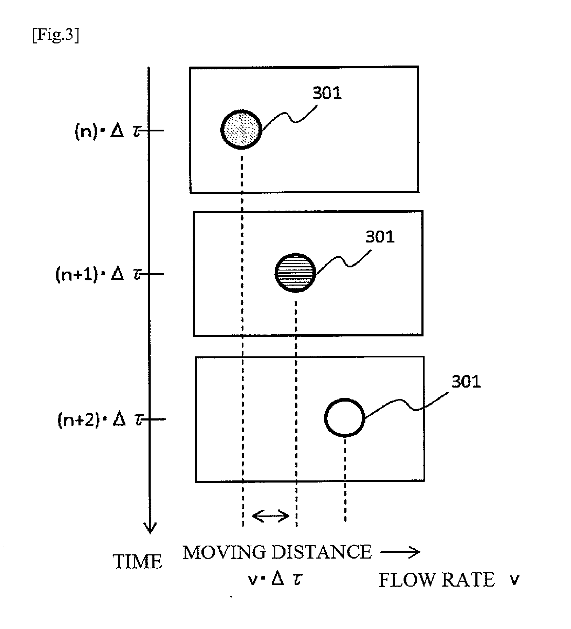

Now, an image processing method used in the present invention will be described with reference to FIG. 3. FIG. 3 is a schematic view showing an example of image processing method.

First, for cell recognition, the cells are numbered when, for example, each cell first appears in the image frame. The cells are managed with the same numbers until disappearing from the image frame. Namely, a state where the cell images move between a plurality of continuous frames is managed by the numbers. In each frame, the cells are sequentially moved downstream such that the cells located upstream are first moved downstream. The cells in the frames are linked under the condition that the moving speed of a specific numbered cell recognized in the images is within a certain range. Owing to this, each cell can be traced with certainty even if the cell is overtaken by another cell. Also owing to this, different monochromatic light components are directed to acquire a plurality of continuous images sequentially, so that absorbed (or transmitted) light spectral imaging of one same cell can be performed.

Thus, cell recognition and absorption spectral imaging can be performed. For numbering the cells, a cell image is first binarized and the center of gravity thereof is found. The luminance center of gravity, area size, circumferential length, longer diameter and shorter diameter of each binarized cell image are found, and the cells are numbered by use of such parameters. Each cell image is automatically stored at this point, because this is advantageous to the user.

Now, in the case where the numbers are used for cell separation, only specific cells need to be separated among the numbered cells. The index for separation may be the above-described information such as the luminance center of gravity, area size, circumferential length, longer diameter and shorter diameter or the like, or information obtained by use of fluorescence as a result of performing fluorescence detection separately from use of the image. In any case, the cells obtained by the detection section are separated in accordance with the numbers. Specifically, the moving speed (V) of each numbered cell is calculated based on the image captured at an interval of a prescribed time duration, and a voltage is applied to each cell at a timing of (L/V) to (L/V+T), where (L) is the distance from the detection to the sorting section, and (T) is the application time duration. A voltage is applied to a cell having a target number when the cell reaches a position between the electrodes. In this manner, the cells are sorted electrically and separated.

Images of a cell 301 flowing in the flow cell at a flow rate v are captured at a certain interval. In this case, as shown in, for example, FIG. 3, a cell image with a first monochromatic light component can be first captured in the n'th image. Then, the next monochromatic pulsed light component is emitted at the timing when the next image frame is to be captured, and thus an image can be captured as the (n+1)th image. During the time duration therebetween, the cell proceeds by v-Δτ in the image in the flow direction. Next, similarly, a third monochromatic pulsed light component is emitted, and an image of the cell at the incident light wavelength can be captured as the (n+2)th image. Again at this point, the cell has proceeded by v·Δτ in the image in the flow direction. These images are moved by v·Δτ in a direction opposite to the flow direction, so that images captured with the monochromatic light components can be layered. In this manner, an absorption characteristic (wavelength dependence of absorption), having a spatial resolution, of a cell or inside of the cell can be acquired by image processing.

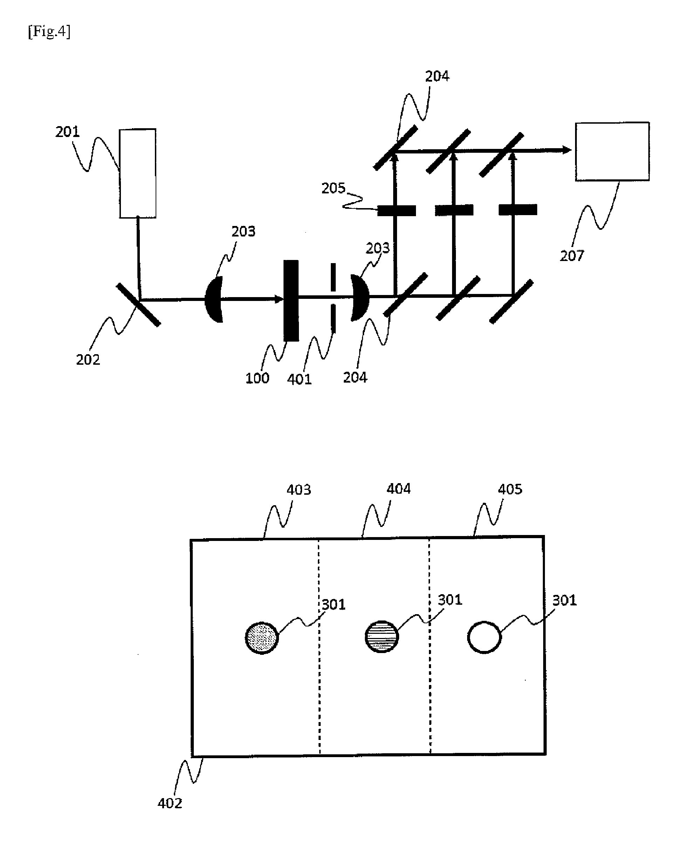

By contrast, in the case where a plurality of wavelengths are processed in one image at the same time, a method shown in, for example, FIG. 4 is effective. An assembly of a plurality of monochromatic pulsed light sources emitting light at the same time, or a while pulsed light source is used as the light source 201. The size of the resultant image is adjusted by use of a slit 401 capable of narrowing an image area. The slit 401 is used so that a light receiving surface of the high speed camera 207 is divided equally into a desired number of spectral areas and the same image is projected thereto, so as to provide spectroscopic imaging. Next, two or more dichroic mirrors and wavelength filters which can equally divide the wavelength range are located side by side. An observation image having a narrowed area is allowed to pass the dichroic mirrors and wavelength filters to subject the image to spectral resolution. Next, the angle of the mirrors 204 are fine-tuned, such that the positions of the light receiving surface of the high speed camera at which the images from the mirrors are projected do not overlap but are located side by side. Owing to such a locating arrangement, substantially the same cell images 403, 404 and 405 of different incident wavelengths can be recorded side by side on one light receiving surface 402. By comparing the light intensities of these cell images located side by side, a spectral distribution of cell images can be acquired.

Now, a method for producing the cell sorter chip 100 used in the present invention will be described.

One example of structure of a cell separation/purification chip is as follows. The cell separation/purification chip includes a series of fine-processed channels located in a two-dimensional manner in a planar chip for performing concentration, arrangement, and purification of cells contained in the sample solution introduced thereto, and means for applying a force on the cells incorporated into the chip.

First, the microchannel 102 is formed inside the chip substrate, and an opening communicating to the channel is provided in a top surface of the chip substrate. The opening is used as a supply opening of samples or necessary buffer solutions (mediums). The channel may be formed by so-called injection molding of causing a plastic material such as PMMA or the like to flow into a die, or by bonding a plurality of glass substrates. The size of the chip is, for example, 50 x 70 x 7 mm (t), but is not limited to this. In order to allow the cells flowing in a groove, through-hole, channel or well formed inside the chip to be observed with a high magnification optical microscope, in the case where a PMMA plastic material is used, a laminate film having a thickness of, for example, 0.1 mm is pressure-contacted by heat; and in the case where glass is used, a glass plate also having a thickness of 0.1 mm is optically bonded. For example, the cells flowing in the channel can be observed through a 0.1 mm-thick laminate film by use of an objective lens of 100 magnifications having a numerical aperture of 1.4. In the case where a plastic material is used, the cells can be observed from above the chip substrate 100 when the plastic material is highly transparent. The cells assumed in the present invention ranges from small bacteria to large animal cells, for example, cancer cells, and also encompass cell populations or cell clusters of such cells. Therefore, the size of the sample cell is typically in a wide range of about 0.5 µm to about 500 µm, but is not strictly limited to this range. Cells of any size are usable as long as the present invention is effectively usable. In the case where cell concentration and cell separation are to be performed successively by use of the channel incorporated into one surface of the substrate, the first issue to consider is the width of the channel (cross-sectional shape). The channel 102 is formed in one of the substrate surfaces in a space of, typically, about 10 to about 100 µm in a thickness direction and thus is formed substantially in a two-dimensional planar shape. In consideration of the size of the cells, an appropriate size of the channel in the thickness direction is about 5 to about 10 µm for bacteria, and is about 100 to about 500 µm for cell populations or cell clusters.

### (Experiment results and screening index)

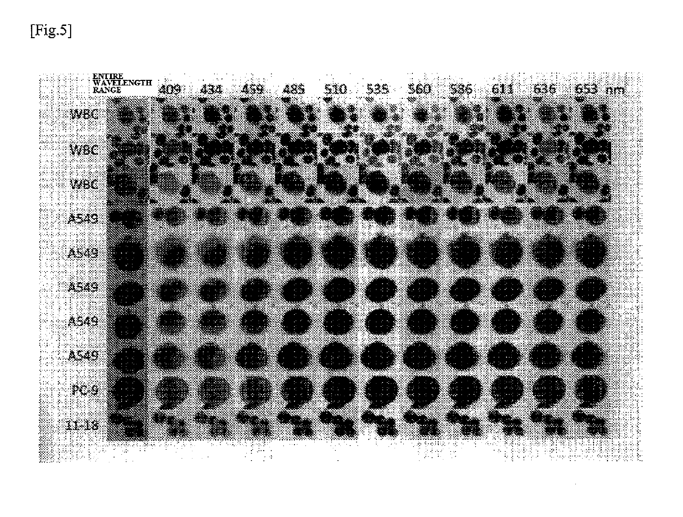

FIG. 5 shows an example of absorption images of various cells actually obtained with various monochromatic light components by use of a cell analysis device in an embodiment according to the present invention. FIG. 5 shows parts of the images obtained at characteristic wavelengths. In this case, the cells are Giemsa-stained. As can be seen from this photograph, the absorption characteristics in the cells are different at each wavelength in accordance with the type of the cell (stain characteristics are different). As described above, by use of such a captured image, information regarding the following can be obtained: (1) presence/absence of the nucleus of the cell or state of the nucleus inside the cell (M-phase), (2) ratio of the size of the nucleus inside the cell with respect to the size of the cell, (3) stain characteristic and the amount of the intracellular organelle at specific absorption wavelengths (or ratio of accumulated area sizes with respect to the cell size), (4) identification of type and number of organelles based on a comparison of absorption characteristics (absorption spectra) of the same position in the cell (same organelle) at a plurality of wavelengths, and (5) whether or not the cells are gathered to form a cluster, characteristics of indexes (1), (2) and (3) mentioned above regarding each cell in the cluster, and the like.

### INDUSTRIAL APPLICABILITY

The present invention is especially useful for separating/purifying trace amounts of target cells in the blood by an index of absorption characteristic of the cell, and for example, analyzing correct genetic information and expression information of the target cells and re-culturing the cells.

### REFERENCE SIGNS LIST

- 1: Cell analysis device
- 100: Cell sorter chip
- 101: Introduction path for sample cell
- 102: Channel
- 103: External force generator
- 104: Direction of external force
- 105: Flow of cell to be recovered (1)
- 106: Flow of cell to be recovered (2)
- 107: Chip substrate
- 108, 109: Branched channel
- 200: Image detection type single-cell separation/purification module
- 201: Laser
- 202: Mirror
- 203: Light collection lens
- 204: Dichroic mirror
- 205: Wavelength filter
- 206: Photomultiplier for fluorescence detection
- 207: High speed camera
- 300: Recording/control section
- 301: Cell
- 401: Slit
- 402: Light receiving surface of the high speed camera
- 403, 404, 405: Cell image obtained with a monochromatic light component

## Claims

1. A cell analysis device for measuring cells in a sample cell solution containing the cells derived from a subject to selectively separate/purify the cells, the cell analysis device comprising:
a cell sorter chip including a first channel for allowing the sample cell solution containing the cells including target cells to flow therein, a cell detection section for detecting an image, obtained by absorption spectral imaging, of the cells in the first channel, and a cell separation section for separating the target cells from the other cells and selectively recovering the target cells, the cell separation section including at least two branched channels branched from the first channel;
an optical system including light directing means for directing light from a plurality of monochromatic light sources located to form a visible light spectrum to the cells flowing in the first channel, and a high speed camera for capturing an image of the cells as an image of each of monochromatic light components at an image capturing rate of at least 200 frames/second, the high speed camera capable of comparing the images of the monochromatic light components to distinguish absorption characteristics of the cells by use of spatial resolution; and
a mechanism for selectively applying, to the cells, an external force for guiding the target cells to either one of the at least two branched channels.

2. The cell analysis device according to claim 1, wherein the external force is an ultrasonic radiation pressure, gravity, an electrostatic force, or a dielectric electrophoretic force.

3. The cell analysis device according to claim 1 or 2, which combines images of the cells obtained at the wavelengths to estimate a visually observed color, detects each of the cells on the level of one cell based on a result of spatial distribution in the cells, and thus identifies the cells to be the target cells.

4. The cell analysis device according to any one of claims 1 through 3, which binarizes an image, obtained by optical imaging, of the entirety of the cells, detects each of the cells on the level of one cell by use of at least one index selected from the group consisting of luminance center of gravity, area size, circumferential length, longer diameter and shorter diameter of the binarized image, and thus identifies the cells to be the target cells.

5. The cell analysis device according to claim 4, wherein the cells in the sample cell solution are labeled with fluorescence, the optical system further includes a fluorescence detector, and information on a fluorescence image of the cells is used as an additional index.

6. A cell analysis device for measuring cells in a sample cell solution containing the cells derived from a subject to detect the cells, the cell analysis device comprising:
a channel chip including a channel for allowing the sample cell solution containing the cells including target cells to flow therein, and a cell detection section for detecting an image, obtained by absorption spectral imaging, of the cells in the channel; and
an optical system including light directing means for directing light from a plurality of monochromatic light sources located to form a visible light spectrum to the cells flowing in the first channel, and a high speed camera for capturing an image of the cells as an image of each of monochromatic light components at an image capturing rate of at least 200 frames/second, the high speed camera capable of comparing the images of the monochromatic light components to distinguish absorption characteristics of the cells by use of spatial resolution.

7. The cell analysis device according to any one of claims 1 through 6, comprising the optical system including the light directing means for directing light from a plurality of monochromatic light sources located to form a visible light spectrum to the cells flowing in the channel, a slit for narrowing a resultant image so as to divide a light receiving surface of the image capturing camera into a number of spectra which are to be acquired, a combination of two or more dichroic mirrors and two or more optical filters capable of equally dividing an image spectrum, and the high speed camera for capturing an image of the cells as an image of each of monochromatic light components at an image capturing rate of at least 200 frames/second, the high speed camera capable of comparing the images of the monochromatic light components to distinguish absorption characteristics of the cells by use of spatial resolution.

8. A cell analyzing method for measuring cells in a sample cell solution containing the cells derived from a subject to selectively separate/purify the cells, the method comprising the steps of:
introducing the sample cell solution into a first channel of a cell sorter chip, which includes the first channel for allowing the sample cell solution containing the cells including target cells to flow therein, a cell detection section for detecting an image, obtained by absorption spectral imaging, of the cells in the first channel, and a cell separation section for separating the target cells from the other cells and selectively recovering the target cells, the cell separation section including at least two branched channels branched from the first channel;
directing light from a plurality of monochromatic light sources to the cells flowing in the first channel;
capturing an image of the cells by use of a high speed camera capable of capturing an image of the cells as an image of each of monochromatic light components at an image capturing rate of at least 200 frames/second and comparing the images of the monochromatic light components to distinguish absorption characteristics of the cells by use of spatial resolution;
selectively applying, to the cells, an external force for guiding the target cells to either one of the at least two branched channels, based on a result of analysis of the captured image; and
recovering the target cells sorted and guided to either one of the branched channels.

9. The method according to claim 8, wherein the cells in the sample cell solution are labeled with an antibody or a dye in advance.

10. The method according to claim 8 or 9, wherein as the sample cell solution, a sample cell solution containing cells sorted by the cell shape and/or staining is used.

11. A cell analysis device for identifying cells in a sample cell solution containing the cells derived from a subject, the cell analysis device comprising:
a pulsed light source including a monochromatic light source array of a plurality of monochromatic light sources corresponding to continuous specific wavelength bands covering a wavelength range from 280 nm to 780 nm, the pulsed light source being controllable by a control program such that each of the monochromatic light sources emits pulsed light;
a cell holding section for holding the sample cell solution containing the cells including target cells, the cell holding section being formed of an optically transparent material to the wavelength range corresponding to the monochromatic light source array;
an image capturing camera having a wavelength characteristic with which an image of the cells can be captured at the wavelength range corresponding to the monochromatic light source array;
a recording section in which the captured image is recorded; and
an image processing section for recording the image in the recording section in a state where a wavelength of each monochromatic light component from the pulsed light source and a frame of the captured image are correlated to each other, and using a result of the recording to compare the images of the monochromatic light components to distinguish absorption characteristics of inside of the cells by use of spatial resolution.

12. The cell analysis device according to claim 11, wherein the pulsed light source includes the array of monochromatic pulsed light sources capable of emitting pulsed light in a time duration which is shorter than an image capturing time duration of one frame of the image capturing camera.

13. The cell analysis device according to claim 12, comprising a high speed camera capable of capturing 10,000 frames per second as the image capturing camera and the array of monochromatic light LED light sources having an emission time less than 0.1 ms.

14. The cell analysis device according to claim 11, which is programmed such that two or more of the monochromatic light sources of the monochromatic light source array emit pulsed light at the same time and thus the image captured by the image capturing camera is an image obtained by absorption of light of any two ore more wavelengths in accordance with the light emission from the monochromatic light sources.

15. The cell analysis device according to claim 11, wherein the cell holding section formed of the optically transparent material to the wavelength range corresponding to the light source for observation is a flow cell having a function of allowing the sample cell solution to flow therein successively.

16. The cell analysis device according to claim 15, wherein the flow cell having the function of allowing the sample cell solution to flow therein successively includes a cell separation/purification section for separating/purifying the cells and moving the target cells downstream with respect to an observation area.

17. The cell analysis device according to claim 11, wherein the order of monochromatic light components directed from the monochromatic light source array is optionally set by the control program.

18. The cell analysis device according to claim 11, which distinguishes the target cells using disappearance of a nucleus in a cell image, obtained from the captured image, as an index.

19. The cell analysis device according to claim 11, which distinguishes the target cells using a ratio between the size of each cell and the size of the nucleus of the corresponding cell in a cell image, obtained from the captured image, as an index.

20. The cell analysis device according to claim 11, which distinguishes the target cells using the degree at which the cells are gathered to form a population in a cell image, obtained from the captured image, as an index.
